# EUROPEAN PATENT APPLICATION

(11) **EP 4 513 190 A1**
(43) Date of publication of application: **26.02.2025**
(21) Application number: 22938667.7
(22) Date of filing: 23.12.2022
(51) Int. Cl.: G01N 33/68, G01N 33/574

(54) **METHOD FOR PREDICTING TREATMENT RESPONSIVENESS TO BIOLOGICAL DRUGS BY MEASURING CONCENTRATION OF ANTI-DRUG ANTIBODIES IN BLOOD**

(30) Priority: 19.04.2022 KR 20220048060
(71) Applicant: Sungkwang Medical Foundation, Seoul 06135 (KR)
(72) Inventor: KIM, Chan, Yongin-si Gyeonggi-do 16903 (KR); CHON, Hong Jae, Seongnam-si Gyeonggi-do 13528 (KR)
(74) Representative: WSL Patentanwälte Partnerschaft mbB
(86) International application number: PCT/KR2022/021222
(87) International publication number: WO 2023/204378

(57) **Abstract**

Disclosed is a method of predicting therapeutic responsiveness to a biological drug by measuring blood concentrations of anti-drug antibodies, wherein the method of predicting therapeutic responsiveness according to an aspect defines a clinically appropriate cut-off value for anti-drug antibody concentration at an initial time point, thereby enabling prediction of therapeutic responsiveness to the biological drug while minimizing false positives. The method provides the benefit of offering data to guide patient treatment decisions and establish appropriate treatment plans.

## Description

### Technical Field

Disclosed is a method of predicting therapeutic responsiveness to a biological drug through the measurement of anti-drug antibody concentration in blood.

### Background Art

Atezolizumab is a humanized IgG1 antibody against programmed death ligand-1 (PD-L1) and demonstrates clinical benefits in various types of cancer. In particular, the combination therapy of atezolizumab and bevacizumab (Atezo/Bev) has completely transformed the treatment landscape of advanced hepatocellular carcinoma (HCC; hereinafter referred to as HCC). This combination therapy demonstrated improved overall survival (OS) compared to sorafenib in the pivotal phase III IMbrave150 trial.

Despite these remarkable advancements, clinical responses to atezolizumab vary widely, ranging from durable complete responses (CR) to rapid disease progression in patients with advanced cancer. To optimize atezolizumab-based immunotherapy, various tumor or circulating factors, including PD-L1 expression, gene expression signatures, and tumor mutational burden (TMB), have been investigated to predict the efficacy of atezolizumab more accurately; however, predictive accuracy remains limited. Recent studies have indicated that serum levels of atezolizumab correlate with clinical outcomes and immune responses.

Administration of humanized antibodies can be immunogenic and may induce undesirable anti-drug antibody (ADA) responses. ADAs can interfere with the action of therapeutic antibodies, affecting drug clearance and serum concentrations, or even neutralizing them. Moreover, a significant reduction in serum concentrations of therapeutic antibodies due to ADAs can impact antitumor efficacy. Several studies have shown that administration of immune checkpoint inhibitors (ICls) can generate ADA responses in cancer patients. In some cases, a subset of patients may possess pre-existing drug-reactive antibodies without prior exposure to ICIs.

Among the various ICls, atezolizumab exhibits the highest ADA incidence rate at 29.8 %, while other ICls show rates of approximately 5 % to 10 %. More importantly, the incidence of neutralizing ADAs that directly block and interfere with the binding of atezolizumab to PD-L1 is 14.5 %. Due to inconsistent findings in previously reported studies, the debate over the clinical significance of atezolizumab ADAs continues. One reason for the mixed results may be that ADA levels can vary over time and can be influenced by various confounding factors such as sampling time, assay format, and patient immune status. In addition, there is a lack of consensus on the cut-off values for defining ADA positivity. It is unclear whether the presence of ADAs at very low concentrations is clinically meaningful, or whether they are only significant when present at concentrations high enough to affect systemic exposure to atezolizumab.

In the IMbrave150 study, 29.6 % of HCC patients developed atezolizumab ADAs at one or more time points after Atezo/Bev treatment. However, data on ADA development patterns outside of clinical trial settings or to guide treatment decisions in patients treated with Atezo/Bev are lacking.

### Disclosure

### Technical Problem

To address the aforementioned issues, an aspect provides a method of predicting therapeutic responsiveness to a biological drug, the method including measuring, from a biological sample isolated from a patient administered with the biological drug, a concentration of anti-drug antibodies specific to the biological drug.

Another aspect provides a diagnostic kit for predicting therapeutic responsiveness to a biological drug, the diagnostic kit including a reagent capable of measuring anti-drug antibodies specific to the biological drug.

### Technical Solution

An aspect provides a method of predicting therapeutic responsiveness to a biological drug, the method including measuring, from a biological sample isolated from a patient administered with the biological drug, a concentration of anti-drug antibodies specific to the biological drug.

In the present specification, the terms "drug", "biological drug" and their plural forms are used interchangeably, and refer to drugs composed of or containing biological molecules or substances (i.e., proteins, polypeptides, peptides, polynucleotides, oligonucleotides, polysaccharides, oligosaccharides and fragments thereof, and cells, tissues, biological fluids, or extracts thereof) which can induce antibodies in a subject. The biological drug may include or target any component involved in molecular and/or cellular processes such as cell cycle, cell survival, apoptosis, immunity, and may be any checkpoint protein or any regulator or inhibitor thereof, or any combination thereof.

In an embodiment, the biological drug may be a biological drug for cancer treatment. The biological drug may be a targeted anticancer agent, an immuno-oncology agent, or a personalized anticancer agent.

In the present specification, the term "targeted anticancer agent" may refer to a drug that selectively attacks only cancer cells by targeting proteins of specific parts of the cancer cells that are different from normal cells. Depending on the type of drug, the targeted anticancer agent may be classified into monoclonal antibody drugs acting on the cell surface and small-molecule compound drugs acting inside the cell. Furthermore, depending on the drug's target of action, the targeted anticancer agent may be classified into EGFR inhibitors, BCR-ABL inhibitors, RAS/MAPK inhibitors, PI3K/AKT/mTOR inhibitors, angiogenesis inhibitors, JAK2 inhibitors, BTK inhibitors, ALK/ROS1 inhibitors, CDK4/6 inhibitors, proteasome inhibitors, CD20 inhibitors, and so on.

In the present specification, the term "immuno-oncology agent" may refer to a therapeutic agent that, unlike conventional anticancer drugs which attack cancer cells directly, stimulates the immune system by introducing artificial immune proteins into the body, thereby inducing immune cells to selectively attack only cancer cells. The immuno-oncology agent may be considered as a drug mechanism that restores or enhances the immune system's tumor recognition or destruction capabilities to overcome the immunosuppressive or immune evasion mechanisms acquired by cancer cells. The immuno-oncology agent may be classified into those used for passive immunotherapy and those used for active immunotherapy. Passive immunotherapy may include, but is not limited to, immune checkpoint inhibitors, immune cell therapies, therapeutic antibodies, etc., and active immunotherapy may include, but is not limited to, cancer vaccines, immune-modulating agents, etc., but is not limited thereto.

In the present specification, the term "immune checkpoint" may also be referred to as "immunological checkpoint" and may refer to molecules that regulate the activity of immune cells. Immune checkpoints exist for self-tolerance to prevent the immune system from indiscriminately attacking cells. The immune checkpoints may be classified into stimulatory checkpoints that enhance immune activity, and inhibitory checkpoints that reduce immune activity. Some cancers utilize inhibitory immune checkpoints to protect themselves from immune responses.

In the present specification, the term "immune checkpoint inhibitor" may refer to a drug that activates immune cells to attack cancer cells by blocking the activity of target immune checkpoint proteins involved in immune cell inhibition. Immune checkpoints that can be targets of immune checkpoint inhibitors may include PD-1, PD-L1, CD80, CD86, CTLA4, B7-H3, -H4, -H5, BTLA, 4-1BB, Tim-3, TIGIT, CD94/NKG2A, KIR2DL-1, -2, -3, etc., and the targets of immune checkpoint inhibitors currently used clinically are PD-1, PD-L1, and CTLA4. More specifically, immune checkpoint inhibitors currently used clinically may include CTLA-4 monoclonal antibodies such as ipilimumab and tremelimumab; PD-1 monoclonal antibodies such as nivolumab, pembrolizumab, and cemiplimab; and PD-L1 monoclonal antibodies such as atezolizumab, durvalumab, and avelumab, but are not limited thereto.

In an embodiment, the biological drug may be an antibody, a protein, a peptide, or a combination thereof. Specifically, the biological drug may include proteins such as monoclonal antibodies, cytokines, soluble receptors, growth factors, hormones, enzymes, adhesion molecules, and fusion proteins and peptides specific to certain targets known to regulate disease mechanisms.

In the present specification, the term "antibody" includes immunoglobulin molecules that immunologically react with specific antigens, and may include both monoclonal antibodies and polyclonal antibodies. In addition, the antibody may include all forms produced by genetic engineering, such as chimeric antibodies (e.g., humanized murine antibodies) and heteroconjugate antibodies (e.g., bispecific antibodies).

In an embodiment, the cancer may be may be urothelial carcinoma (UC), non-small cell lung cancer, small cell lung cancer, triple-negative breast cancer (TNBC), hepatocellular carcinoma, colorectal cancer, lung cancer, glioblastoma multiforme, renal cell carcinoma, melanoma, bladder cancer, classical Hodgkin lymphoma (CHL), esophageal squamous cell carcinoma, esophageal cancer, gastric cancer, or head and neck squamous cell carcinoma.

In an embodiment, the antibody may be an immune checkpoint inhibitor, a targeted anticancer agent, or a combination thereof.

*20In an embodiment, the immune checkpoint inhibitor may be a CTLA4 inhibitor, a PD-L1 inhibitor, a PD-1 inhibitor, or a combination thereof. The immune checkpoint inhibitor may be ipilimumab, tremelimumab, pembrolizumab, cemiplimab, nivolumab, durvalumab, atezolizumab, avelumab, or a combination thereof. The targeted anticancer agent may be cetuximab, trastuzumab, ibritumomab, rituximab, brentuximab, alemtuzumab, bevacizumab, panitumumab, or a combination thereof.

In an embodiment, the patient may be a cancer patient and may be a patient undergoing combination therapy with atezolizumab and bevacizumab.

In an embodiment, the biological sample may be one or more selected from the group consisting of blood cells, blood, serum, plasma, bone marrow, lymph fluid, urine, sputum, saliva, feces, semen, spinal cord, or cerebrospinal fluid (CSF);
external secretions of the skin, respiratory tract, gastrointestinal tract, and genitourinary tracts;
tears, milk, any human organ or tissue, any sample obtained by lavage, optionally from the breast ductal system, pleural effusion, in vitro or ex vivo cell cultures, and samples of cell culture components.

In an embodiment, the biological sample may be one that is isolated after 14 days to 28 days, 14 days to 27 days, 14 days to 26 days, 14 days to 25 days, 14 days to 24 days, 14 days to 23 days, 14 days to 22 days, 14 days to 21 days, 14 days to 20 days, 14 days to 19 days, 14 days to 18 days, 15 days to 28 days, 15 days to 27 days, 15 days to 26 days, 15 days to 25 days, 15 days to 24 days, 15 days to 23 days, 15 days to 22 days, 15 days to 21 days, 15 days to 20 days, 15 days to 19 days, 15 days to 18 days, 16 days to 28 days, 16 days to 27 days, 16 days to 26 days, 16 days to 25 days, 16 days to 24 days, 16 days to 23 days, 16 days to 22 days, 16 days to 21 days, 16 days to 20 days, 16 days to 19 days, 16 days to 18 days, 17 days to 28 days, 17 days to 27 days, 17 days to 26 days, 17 days to 25 days, 17 days to 24 days, 17 days to 23 days, 17 days to 22 days, 17 days to 21 days, 17 days to 20 days, 17 days to 19 days, 17 days to 18 days, 18 days to 28 days, 18 days to 27 days, 18 days to 26 days, 18 days to 25 days, 18 days to 24 days, 18 days to 23 days, 18 days to 22 days, 18 days to 21 days, 18 days to 20 days, 18 days to 19 days, 19 days to 28 days, 19 days to 27 days, 19 days to 26 days, 19 days to 25 days, 19 days to 24 days, 19 days to 23 days, 19 days to 22 days, 19 days to 21 days, 19 days to 20 days, 20 days to 28 days, 20 days to 27 days, 20 days to 26 days, 20 days to 25 days, 20 days to 24 days, 20 days to 23 days, 20 days to 22 days, 20 days to 21 days, 21 days to 28 days, 21 days to 27 days, 21 days to 26 days, 21 days to 25 days, 21 days to 24 days, 21 days to 23 days, 21 days to 22 days, or after 21 days have passed following initiation of treatment with the patient.

The biological drug is known to induce in vivo formation of "anti-drug antibodies (ADAs)" in some cases, and their detection has generally become equivalent to a measure of immunogenicity. Most adverse effects due to ADA formation, such as pharmacological abrogation, effects on therapeutic exposure, or hypersensitivity reactions, are the result of the formation of immune complexes between ADAs and therapeutic proteins. Their levels, dynamics of interactions, size, polyclonal diversity, distribution, and Fc-mediated physiological effects may potentially translate into clinically observable adverse effects. ADAs are generally polyclonal and may include different isotypes [immunoglobulin (Ig)G, IgA, IgM, or IgE], bind to different regions ('domains') of the drug molecule, have varying affinities (binding strengths), and may vary between patients, thus representing a very complex set of analytes.

In the present specification, the term "anti-drug antibodies" or "ADAs" refers to antibodies that bind to a therapeutic agent and may affect the serum concentration and function of the therapeutic agent in an individual. The presence of ADAs may increase the loss of a therapeutic agent through the formation of immune complexes between the therapeutic agent and antibodies (neutralizing, non-neutralizing, or both), thereby reducing the half-life of the therapeutic agent. Furthermore, the activity and usefulness of the therapeutic agent may be reduced through antibodies binding to the therapeutic agent (especially in the case of neutralizing ADAs). ADAs may also be associated with other adverse effects such as allergic or hypersensitivity reactions and neutralization of host proteins.

In an embodiment, the anti-drug antibodies may be an anti-ipilimumab antibody, an anti-tremelimumab antibody, an anti-pembrolizumab antibody, an anti-cemiplimab antibody, an anti-nivolumab antibody, an anti-durvalumab antibody, an anti-atezolizumab antibody, an anti-avelumab antibody, an anti-cetuximab antibody, an anti-trastuzumab antibody, an anti-ibritumomab antibody, an anti-rituximab antibody, an anti-brentuximab antibody, an anti-alemtuzumab antibody, an anti-bevacizumab antibody, an anti-panitumumab antibody, or a combination thereof.

In an embodiment, a concentration of the anti-drug antibodies may be measured by one or more methods selected from the group consisting of western blotting, radioimmunoassay (RIA), radioimmunodiffusion, enzyme-linked immunosorbent assay (ELISA), immunoprecipitation, flow cytometry, immunofluorescence, ouchterlony, complement fixation assay, and protein chip, but is not limited thereto and may be measured according to conventional methods known in the art.

In an embodiment, determining therapeutic responsiveness to the biological drug to be low when the measured concentration of anti-drug antibodies is equal to or greater than a cut-off value may be further included. It can be predicted that during drug treatment, patients whose anti-drug antibody concentration is equal to or greater than the cut-off value will have decreased systemic exposure to the drug and show a reduction in the drug's immunotherapeutic efficacy, compared to patients with concentrations below the cut-off value.

In the present specification, the term "cut-off value" refers to a numerical value used in mediating classification of two or more states (e.g., high therapeutic responsiveness and low therapeutic responsiveness to immuno-oncology agents in hepatocellular carcinoma patients) for a biological sample. For example, when a given parameter is equal to or less than the cut-off value, the first classification of quantitative data (e.g., cases with high therapeutic responsiveness to immuno-oncology agents in hepatocellular carcinoma patients) is made, or when the parameter exceeds the cut-off value, another classification of quantitative data (e.g., cases with low therapeutic responsiveness to immuno-oncology agents in hepatocellular carcinoma patients) is made.

In the present specification, the term "PFS" or "progression-free survival" refers to the period during which a patient survives without progression of the disease until the patient experiences worsening or death, and may be a method for observing whether a new treatment is effective. The term "progression" may refer to an increase in tumor size by 20% or more.

In the present specification, the term "OS" or "overall survival" refers to the time from random assignment to death, and may refer to the numerical value tracking the period from when the patient starts treatment until the moment of death.

In an embodiment, the cut-off value may be 500 ng/mL to 2,000 ng/mL, 500 ng/mL to 1,900 ng/mL, 500 ng/mL to 1,800 ng/mL, 500 ng/mL to 1,700 ng/mL, 500 ng/mL to 1,600 ng/mL, 500 ng/mL to 1,500 ng/mL, 500 ng/mL to 1,400 ng/mL, 500 ng/mL to 1,300 ng/mL, 500 ng/mL to 1,200 ng/mL, 500 ng/mL to 1,100 ng/mL, 500 ng/mL to 1,000 ng/mL, 500 ng/mL to 900 ng/mL, 500 ng/mL to 800 ng/mL, 500 ng/mL to 700 ng/mL, 500 ng/mL to 600 ng/mL, 600 ng/mL to 2,000 ng/mL, 600 ng/mL to 1,900 ng/mL, 600 ng/mL to 1,800 ng/mL, 600 ng/mL to 1,700 ng/mL, 600 ng/mL to 1,600 ng/mL, 600 ng/mL to 1,500 ng/mL, 600 ng/mL to 1,400 ng/mL, 600 ng/mL to 1,300 ng/mL, 600 ng/mL to 1,200 ng/mL, 600 ng/mL to 1,100 ng/mL, 600 ng/mL to 1,000 ng/mL, 600 ng/mL to 900 ng/mL, 600 ng/mL to 800 ng/mL, 600 ng/mL to 700 ng/mL, 700 ng/mL to 2,000 ng/mL, 700 ng/mL to 1,900 ng/mL, 700 ng/mL to 1,800 ng/mL, 700 ng/mL to 1,700 ng/mL, 700 ng/mL to 1,600 ng/mL, 700 ng/mL to 1,500 ng/mL, 700 ng/mL to 1,400 ng/mL, 700 ng/mL to 1,300 ng/mL, 700 ng/mL to 1,200 ng/mL, 700 ng/mL to 1,100 ng/mL, 700 ng/mL to 1,000 ng/mL, 700 ng/mL to 900 ng/mL, 700 ng/mL to 800 ng/mL, 800 ng/mL to 2,000 ng/mL, 800 ng/mL to 1,900 ng/mL, 800 ng/mL to 1,800 ng/mL, 800 ng/mL to 1,700 ng/mL, 800 ng/mL to 1,600 ng/mL, 800 ng/mL to 1,500 ng/mL, 800 ng/mL to 1,400 ng/mL, 800 ng/mL to 1,300 ng/mL, 800 ng/mL to 1,200 ng/mL, 800 ng/mL to 1,100 ng/mL, 800 ng/mL to 1,000 ng/mL, 800 ng/mL to 900 ng/mL, 900 ng/mL to 2,000 ng/mL, 900 ng/mL to 1,900 ng/mL, 900 ng/mL to 1,800 ng/mL, 900 ng/mL to 1,700 ng/mL, 900 ng/mL to 1,600 ng/mL, 900 ng/mL to 1,500 ng/mL, 900 ng/mL to 1,400 ng/mL, 900 ng/mL to 1,300 ng/mL, 900 ng/mL to 1,200 ng/mL, 900 ng/mL to 1,100 ng/mL, 900 ng/mL to 1,000 ng/mL, 1,000 ng/mL to 2,000 ng/mL, 1,000 ng/mL to 1,900 ng/mL, 1,000 ng/mL to 1,800 ng/mL, 1,000 ng/mL to 1,700 ng/mL, 1,000 ng/mL to 1,600 ng/mL, 1,000 ng/mL to 1,500 ng/mL, 1,000 ng/mL to 1,400 ng/mL, 1,000 ng/mL to 1,300 ng/mL, 1,000 ng/mL to 1,200 ng/mL, 1,000 ng/mL to 1,100 ng/mL, 1,100 ng/mL to 2,000 ng/mL, 1,100 ng/mL to 1,900 ng/mL, 1,100 ng/mL to 1,800 ng/mL, 1,100 ng/mL to 1,700 ng/mL, 1,100 ng/mL to 1,600 ng/mL, 1,100 ng/mL to 1,500 ng/mL, 1,100 ng/mL to 1,400 ng/mL, 1,100 ng/mL to 1,300 ng/mL, 1,100 ng/mL to 1,200 ng/mL, 1,200 ng/mL to 2,000 ng/mL, 1,200 ng/mL to 1,900 ng/mL, 1,200 ng/mL to 1,800 ng/mL, 1,200 ng/mL to 1,700 ng/mL, 1,200 ng/mL to 1,600 ng/mL, 1,200 ng/mL to 1,500 ng/mL, 1,200 ng/mL to 1,400 ng/mL, 1,200 ng/mL to 1,300 ng/mL, 1,300 ng/mL to 2,000 ng/mL, 1,300 ng/mL to 1,900 ng/mL, 1,300 ng/mL to 1,800 ng/mL, 1,300 ng/mL to 1,700 ng/mL, 1,300 ng/mL to 1,600 ng/mL, 1,300 ng/mL to 1,500 ng/mL, 1,300 ng/mL to 1,400 ng/mL, or 1,000 ng/mL.

In the present specification, the term "therapeutic responsiveness" may refer to whether a specific drug has a therapeutic effect on an individual patient's disease.

In the present specification, the term "predict" may mean pre-determining a specific result, for example, therapeutic responsiveness, by identifying characteristics, such as the concentration of anti-drug antibodies specific to a biological drug in a biological sample.

In the present specification, the term "prediction of therapeutic responsiveness in cancer patients" may refer to predicting in advance whether the administration of a drug may be useful in the treatment of cancer before administration and may involve predicting therapeutic responsiveness to the drug by measuring the expression levels of genes, proteins, antibodies, or metabolites.

Another aspect provides a diagnostic kit for predicting therapeutic responsiveness to a biological drug, the diagnostic kit including a reagent capable of measuring anti-drug antibodies specific to a biological drug.

The meanings of the biological drug and anti-drug antibody are as described above.

In an embodiment, the subject whose therapeutic responsiveness is to be predicted may be a patient who has passed, after starting treatment with the biological drug, 14 days to 28 days, 14 days to 27 days, 14 and 26 days, 14 days to 25 days, 14 days to 24 days, 14 days to 23 days, 14 days to 22 days, 14 days to 21 days, 14 days to 20 days, 14 days to 19 days, 14 days to 18 days, 15 days to 28 days, 15 days to 27 days, 15 days to 26 days, 15 days to 25 days, 15 days to 24 days, 15 days to 23 days, 15 days to 22 days, 15 days to 21 days, 15 days to 20 days, 15 days to 19 days, 15 days to 18 days, 16 days to 28 days, 16 days to 27 days, 16 days to 26 days, 16 days to 25 days, 16 days to 24 days, 16 days to 23 days, 16 days to 22 days, 16 days to 21 days, 16 days to 20 days, 16 days to 19 days, 16 days to 18 days, 17 days to 28 days, 17 days to 27 days, 17 days to 26 days, 17 days to 25 days, 17 days to 24 days, 17 days to 23 days, 17 days to 22 days, 17 days to 21 days, 17 days to 20 days, 17 days to 19 days, 17 days to 18 days, 18 days to 28 days, 18 days to 27 days, 18 days to 26 days, 18 days to 25 days, 18 days to 24 days, 18 days to 23 days, 18 days to 22 days, 18 days to 21 days, 18 days to 20 days, 18 days to 19 days, 19 days to 28 days, 19 days to 27 days, 19 days to 26 days, 19 days to 25 days, 19 days to 24 days, 19 days to 23 days, 19 days to 22 days, 19 days to 21 days, 19 days to 20 days, 20 days to 28 days, 20 days to 27 days, 20 days to 26 days, 20 days to 25 days, 20 days to 24 days, 20 days to 23 days, 20 days to 22 days, 20 days to 21 days, 21 days to 28 days, 21 days to 27 days, 21 days to 26 days, 21 days to 25 days, 21 days to 24 days, 21 days to 23 days, 21 days to 22 days, or 21 days.

The patient may be a cancer patient and may be a patient undergoing combination therapy with atezolizumab and bevacizumab.

In an embodiment, the reagent capable of measuring anti-drug antibodies may be an antibody. The anti-drug antibodies are as described above.

In an embodiment, the biological drug may be a biological drug for treating a cancer. The cancer may be urothelial carcinoma (UC), non-small cell lung cancer, small cell lung cancer, triple-negative breast cancer (TNBC), hepatocellular carcinoma, colorectal cancer, lung cancer, glioblastoma multiforme, renal cell carcinoma, melanoma, bladder cancer, classical Hodgkin lymphoma (CHL), esophageal squamous cell carcinoma, esophageal cancer, gastric cancer, or head and neck squamous cell carcinoma.

In an embodiment, the biological drug may be an antibody, a protein, a peptide, or a combination thereof. The meaning of the antibody is as described above.

In an embodiment, the antibody may be an immune checkpoint inhibitor, a targeted anticancer agent, or a combination thereof. The details regarding the immune checkpoint inhibitor and targeted anticancer agent are as described above.

In an embodiment, the therapeutic responsiveness to the biological drug may be determined to be low when the concentration of anti-drug antibodies is equal to or greater than a cut-off value. The details regarding this determination are as described above.

The meaning of the cut-off value is as described above.

### Advantageous Effects

A method of predicting therapeutic responsiveness according to an aspect defines a clinically appropriate cut-off value for anti-drug antibody concentration at an initial time point, thereby enabling prediction of immune therapeutic responsiveness to a biological drug while minimizing false positives. This may provide the benefit of offering data to guide patient treatment decisions and establish appropriate treatment plans.

### Description of Drawings

FIG. 1 is a Consolidated Standards of Reporting Trials (CONSORT) diagram for a hepatocellular carcinoma (HCC) cohort study.

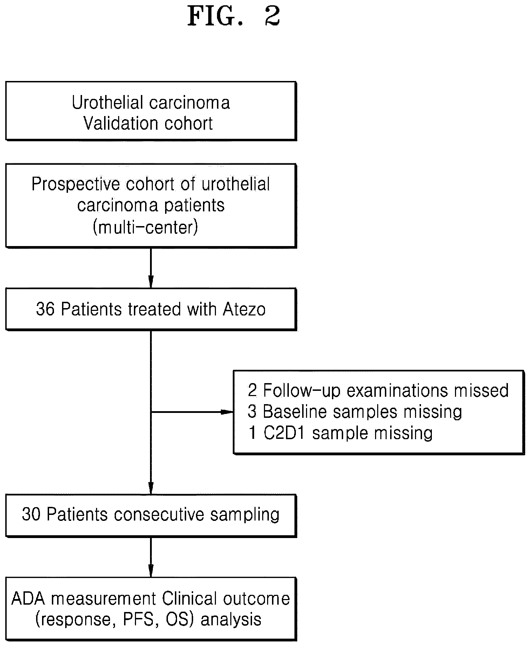

FIG. 2 is a Consolidated Standards of Reporting Trials (CONSORT) diagram for a urothelial carcinoma (UC) cohort study.
FIG. 3 is a graph comparing anti-drug antibody (ADA) levels between baseline and cycle 2 day 1 (C2D1) in a discovery cohort.

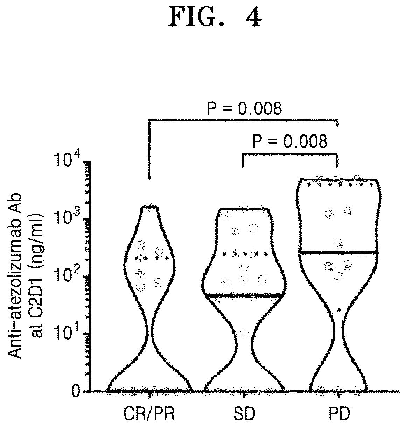

FIG. 4 is a graph comparing ADA levels at C2D1 according to best response in a discovery cohort.
FIG. 5 is a graph comparing ADA levels between baseline and C2D1 in a validation cohort.
FIG. 6 is a graph comparing ADA levels at C2D1 according to best response in a validation cohort.
FIG. 7 is an integrated analysis graph of ADA levels at baseline and C2D1.
FIG. 8 is a graph showing best response, progression-free survival (PFS), and overall survival (OS) according to ADA levels in a discovery cohort (HCC, n = 50).
FIG. 9 is a graph showing progression-free survival (PFS) according to ADA levels in a discovery cohort (HCC, n = 50).
FIG. 10 is a graph showing overall survival (OS) according to ADA levels in a discovery cohort (HCC, n = 50).
FIG. 11 is a graph showing best response according to ADA levels in an independent multicentric validation cohort (HCC, n = 82).
FIG. 12 is a graph showing progression-free survival (PFS) according to ADA levels in an independent multicentric validation cohort (HCC, n = 82).
FIG. 13 is a graph showing overall survival (OS) according to ADA levels in an independent multicentric validation cohort (HCC, n = 82).
FIG. 14 is a graph showing best response, progression-free survival (PFS), and overall survival (OS) according to ADA levels in a UC validation cohort (n = 30).
FIG. 15 is a meta-analysis forest plot showing PFS according to age, sex, Eastern Cooperative Oncology Group (ECOG) performance status, Child-Pugh score, alpha-fetoprotein (AFP), macrovascular invasion (MVI), extrahepatic spread, neutrophil-to-lymphocyte ratio (NLR), and ADA status.
FIG. 16 is a meta-analysis forest plot showing OS according to age, sex, ECOG performance status, Child-Pugh score, alpha-fetoprotein(AFP), macrovascular invasion(MVI), extrahepatic spread, neutrophil-to-lymphocyte ratio(NLR), and ADA status.
FIG. 17 is a meta-analysis forest plot showing PFS according to various cut-offs for ADA positivity.
FIG. 18 is a meta-analysis forest plot showing OS according to various cut-offs for ADA positivity.
FIG. 19 is a receiver operating curve (AUC 0.78, 95 % Cl, 0.63-0.82) for 12-month OS.
FIG. 20 is a graph comparing serum atezolizumab concentrations according to ADA levels.
FIG. 21 is a graph showing a correlation between serum atezolizumab and ADA concentrations through Pearson correlation analysis. (Solid line: regression curve; Dashed lines: 95 % confidence interval bands)
FIG. 22 is a representative flow cytometry plot and comparative graph of Ki67+ proliferating CD8+ T cells between baseline and C2D1 according to ADA status.
FIG. 23 is a representative flow cytometry plot and comparative graph of intracellular interferon-γ and tumor necrosis factor-α from CD8+ T cells according to ADA status.

### Mode for Invention

The present disclosure will be described in greater detail through the following examples below. However, these examples are provided for illustrative purposes, and the scope of the present disclosure is not limited to these examples.

### Reference Example 1. Patients and Methods

The aim was to discover the clinical impact of ADA in prospectively enrolled patients who were diagnosed with advanced hepatocellular carcinoma (HCC) at CHA Bundang Medical Center in Korea (discovery cohort) and received Atezo/Bev treatment. Patient enrollment was expanded to two independent cohorts: patients with advanced hepatocellular carcinoma (validation cohort) and patients with advanced urothelial carcinoma (UC validation cohort). These patients were enrolled at four independent tertiary cancer centers in Korea (Bundang Medical Center, Ulsan University Hospital, Haeundae Paik Hospital, and St. Louis).

The eligibility criteria for the HCC discovery and validation cohorts were: age 20 years or older; locally advanced or unresectable HCC confirmed by histological or cytological analysis or by clinical features according to the American Association for the Study of Liver Diseases cirrhosis patient criteria; no prior systemic therapy; Child-Pugh class A; and an Eastern Cooperative Oncology Group (ECOG) score of 0 to 1.

The eligibility criteria for the UC validation cohort were: age 20 years or older; locally advanced or metastatic UC of the urinary tract confirmed by histological or cytological analysis; during or after platinum-based chemotherapy; and an ECOG score of 0 to 2.

This study was approved by the Institutional Review Boards (IRBs) of the relevant institutions (CHA Bundang Medical Center, CHA-2017-11-052, CHA-2017-11-054; Ulsan University Hospital, 2020-12-006; Haeundae Paik Hospital, 2020-12-019-001; St. Vincent's Hospital, VC16TISI0208). All patients provided written informed consent.

### Reference Example 2. Treatment and Evaluation

All patients in the HCC discovery and validation cohorts were treated with a combination of atezolizumab (fixed dose of 1,200 mg) and bevacizumab (15 mg/kg) every three weeks. Patients in the UC validation cohort were treated with atezolizumab (fixed dose of 1,200 mg) every three weeks. Treatment interruptions or dose reductions were made according to the protocols of the IMbrave 150 or IMvigor 210 trials. Treatment was continued until intolerable toxicity, disease progression, or patient withdrawal of consent occurred. Responses were evaluated according to RECIST 1.1. Response evaluations were performed every 6 or 9 weeks using computed tomography or magnetic resonance imaging.

### Reference Example 3. Sample Collection and ADA Quantification

Blood samples were collected in Vacutainer tubes (BD Biosciences) before the first administration of atezolizumab (cycle 1 day 1, hereinafter referred to as baseline) and before the second injection of atezolizumab (cycle 2 day 1, hereinafter referred to as C2D1). Samples were left to coagulate, and serum was centrifuged at 1,000 Xg for 5 minutes and stored at -80 °C. Peripheral blood mononuclear cells were isolated by density gradient centrifugation using Ficoll-Paque PLUS (GE Healthcare) and preserved in liquid nitrogen. C2D1 blood samples collected before 18 days after the first treatment initiation or after 24 days or more were excluded from the analysis to ensure consistent timing.

### Reference Example 4. Measurement of Anti-Atezolizumab Antibody and Atezolizumab

The concentration of anti-atezolizumab antibodies was measured using a commercial enzyme-linked immunosorbent assay (ELISA; KBI2027 ver 4.0, KRISHGEN BioSystems) according to the manufacturer's instructions. In brief, samples were incubated at 37 °C for 120 minutes in micro-wells pre-coated with atezolizumab. After multiple washes, horseradish peroxidase (HRP)-conjugated atezolizumab was added, and the plate was incubated at 37 °C for 60 minutes. After another wash, TMB substrate solution was added, and the plate was incubated in the dark at 37 °C for 30 minutes. The absorbance of each well was measured at 450 nm using a microplate reader (Bio Tek). The serum concentration of atezolizumab was measured using a commercial ELISA assay (KBI1027, KRISHGEN BioSystems) according to the manufacturer's instructions.

### Reference Example 5. Cytokine Secretion Analysis and Flow Cytometry

To evaluate cytokine secretion, peripheral blood mononuclear cells were stimulated with plate-bound anti-CD3 antibody (1 µg/ml). After 4 hours, brefeldin A (eBioscience) and monensin (eBioscience) were added to the culture medium. After 20 hours, the activated cells were harvested. To investigate the proliferation ability of CD8+ T cells, cryopreserved peripheral blood mononuclear cells were used after thawing. Dead cells were excluded by using Fixable Viability Dye eFluor^{™} 780 (eBioscience) on ice for 30 minutes before antibody staining, followed by treatment with human Fc receptor binding inhibitor (eBioscience) at room temperature for 15 minutes. Surface proteins were stained on ice for 30 minutes with fluorochrome-conjugated antibodies; anti-human CD3 antibody (clone SK7, eBioscience), anti-human CD4 antibody (clone RPA-T4, BioLegend), and anti-human CD8 antibody (clone RPA-T8, eBioscience).

For intracellular staining, cells were fixed and permeabilized using the FoxP3 staining buffer kit (Thermo Fisher Scientific). Then, cells were stained with fluorochrome-conjugated antibodies; anti-human Ki-67 antibody (clone Ki-67, BioLegend), anti-human interferon-γ antibody (clone B27, BD Bioscience), and anti-human tumor necrosis factor-α antibody (clone Mab11, BD Bioscience).

Flow cytometry was performed using a CytoFLEX flow cytometer (Beckman Coulter), and the results were analyzed using FlowJo software (Tree Star Inc., Ashland, OR, USA).

### Reference Example 6. Statistical Analysis

For comparison of variables, unpaired t-test or paired t-test, ANOVA, and chi-square test were used. OS was defined as the time from the start of treatment to death from any cause. Progression-free survival (PFS) was defined as the time between the start of treatment and disease progression or death from any cause. Survival outcomes were analyzed using the Kaplan-Meier method, and subgroups were compared using the log-rank test. Univariate and multivariate analyses of OS and PFS were performed using the Cox proportional hazards model. ROC curves were plotted using R 4.1.2. Spearman correlation was used to relate serum ADA and atezolizumab. Statistical significance was defined as two-sided P < 0.05. Statistical analyses were performed using SPSS version 18.0 (IBM, NY, USA).

### Example 1. Baseline Demographic Analysis of Hepatocellular Carcinoma (HCC) Patients and Urothelial Carcinoma (UC) Patients

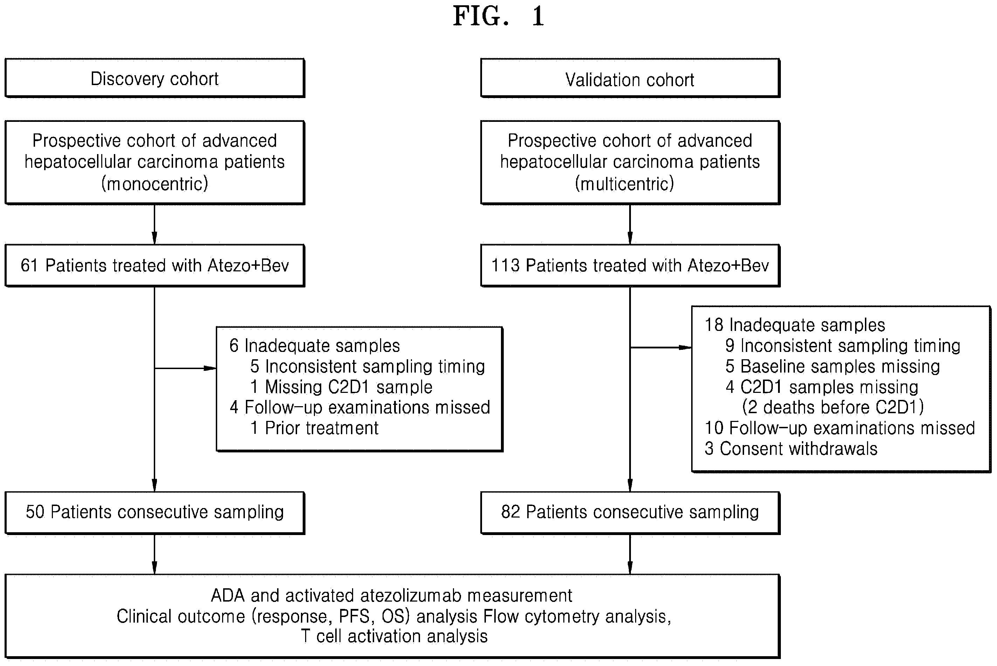

From June 2020 to July 2021, a total of 132 patients with advanced hepatocellular carcinoma who were treated with first-line Atezo/Bev were prospectively enrolled in the discovery cohort (50 patients from a single center) and the validation cohort (82 patients from four centers). For the UC validation cohort, 30 patients with advanced UC treated with atezolizumab between November 2019 and July 2021 were enrolled. The CONSORT diagram for the study is shown in FIG. 1.

The baseline characteristics of patients with advanced hepatocellular carcinoma are shown in Table 1.

**[Table 1]**

| | Total HCC cohort (N=132) | Discovery cohort (N=50) | Validation cohort (n=82) |
|---|---|---|---|
| Median age (interquartile range, IQR) | 61 (55-69) | 61 (55-70) | 61 (53-68) |
| Male sex | 111 (84.1%) | 41 (82.0%) | 70 (85.4%) |
| ECOG performance status | | | |
| 0 | 71 (53.8%) | 25 (50.0%) | 46 (56.1%) |
| 1 | 61 (46.2%) | 25 (50.0%) | 36 (43.9%) |
| Child-Pugh classification | | | |
| A5 | 86 (65.2%) | 35 (70.0%) | 51 (62.2%) |
| A6 | 46 (34.8%) | 15 (30.0%) | 31 (37.8%) |
| Barcelona Clinical liver cancer stage | | | |
| B | 24 (18.2%) | 10 (20.0%) | 14 (17.1%) |
| C | 108 (81.8%) | 40 (80.0%) | 68 (82.9%) |
| Alpha-fetoprotein ≥ 400 ng/ml, n (%) | 42 (31.8%) | 16 (32.0%) | 26 (31.7%) |
| Neutrophil to lymphocyte ratio (IQR) | 2.6 (1.7-4.2) | 2.7 (1.6-4.4) | 2.6 (1.8-4.0) |
| Presence of macrovascular invasion | 55 (41.7%) | 20 (40.0%) | 35 (42.7%) |
| Presence of extrahepatic spread | 74 (56.1%) | 28 (56.0%) | 46 (56.1%) |
| Etiology of HCC | | | |
| Hepatitis B | 89 (67.4%) | 34 (68.0%) | 55 (67.1%) |
| Hepatitis C | 7 (5.3%) | 4 (8.0%) | 3 (3.7%) |
| Alcohol | 21 (15.9%) | 8 (16.0%) | 13 (15.9%) |
| Other or unknown | 15 (11.4%) | 4 (8.0%) | 11 (13.4%) |
| Prior local therapy for HCC | 81 (61.4%) | 34 (68.0%) | 47 (57.3%) |
| Median ADA (IQR) at baseline | 0 (0-0) | 0 (0-0) | 0 (0-0) |
| Median ADA (IQR) at C2D1 | 45.95(0-257.9) | 77.45(0-356.0) | 33.55(0-193.1) |
| Anti-drug antibody at C2D1 | | | |
| Negative or low (<1000 ng/ml) | 109 (82.6%) | 41 (82.0%) | 68 (82.9%) |
| High (≥1000 ng/ml) | 23 (17.4%) | 9 (18.0%) | 14 (17.1%) |

The mean age was 61 years, and males comprised 84.1%. Most patients were Child-Pugh class A6 and BCLC stage C. Hepatitis B virus infection was the most common cause of HCC. The discovery and validation cohorts had similar clinical and laboratory characteristics. In the discovery cohort, the objective response rate (ORR) of was 30.0 %, the 1-year OS rate was 66.6 %, and the median follow-up period was 19.4 months. In the validation cohort, the ORR was 25.6 %, the 1-year OS rate was 65.2 %, and the median follow-up period was 13.4 months.

The baseline characteristics of patients with advanced urothelial carcinoma are shown in Table 2 below.

**[Table 2]**

| | Validation cohort UC (N=30) |
|---|---|
| Median age (interquartile range) | 68 (63-76) |
| Male sex, n (%) | 25 (83.3) |
| ECOG performance status, n (%) | |
| 0 | 12 (40.0) |
| 1 | 13 (43.3) |
| 2 | 5 (16.7) |
| Tumor site, n (%) | |
| Bladder | 15 (50.0) |
| Ureter | 10 (33.3) |
| Renal pelvis | 5 (16.7) |
| Sites of metastasis, n (%) | |
| Visceral | 27 (90.0) |
| Liver | 8 (26.7) |
| Lymph node only | 2 (6.7) |
| Previous therapy with platinum, n (%) | |
| Cisplatin-based | 29 (96.7) |
| Carboplatin-based | 1 (3.3) |
| Number of previous systemic therapy in the metastatic setting, n (%) | |
| 1 | 25 (83.3) |
| 2 | 4 (13.3) |
| 3 | 1 (3.3) |
| Number by Bellmunt risk score, n (%) | |
| 0 | 3 (10.0) |
| 1 | 7 (23.3) |
| 2 | 9 (30.0) |
| ≥ 3 | 11 (36.7) |
| Anti-drug antibody at cycle 2 day 1, n (%) | |
| Negative or low (<1000 ng/ml) | 26 (83.7) |
| High (≥1000 ng/ml) | 4 (13.3) |

The mean age was 68 years, and males comprised 83.3 %. The most common tumor site was the bladder (50.0 %), and most patients (90.0 %) had visceral metastases. All patients in the UC validation cohort were treated with atezolizumab after failure of prior platinum-based therapy, with an ORR of 13.3 %, a median OS of 9.0 months, and a median follow-up period of 5.9 months.

### Example 2. Comparison of Serum Atezolizumab ADA Levels at Baseline and C2D1 (Cycle 2 Day 1)

To compare the serum atezolizumab ADA levels at baseline and C2D1, the methods described in Reference Examples 1 to 4 and Reference Example 6 were performed.

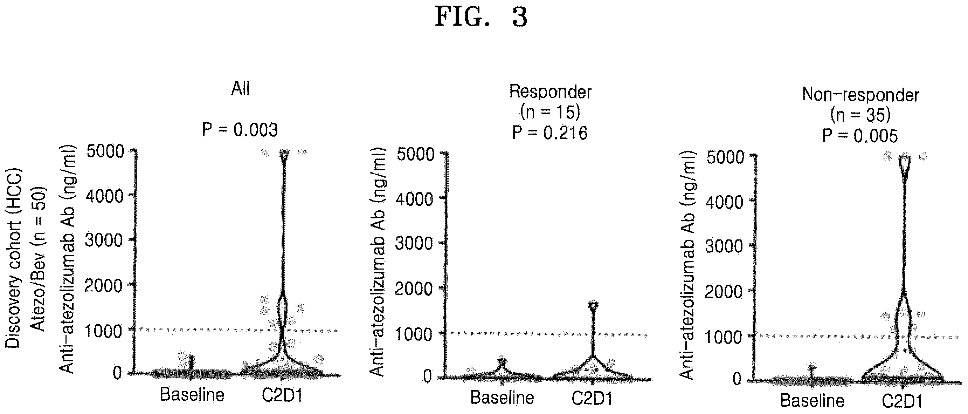

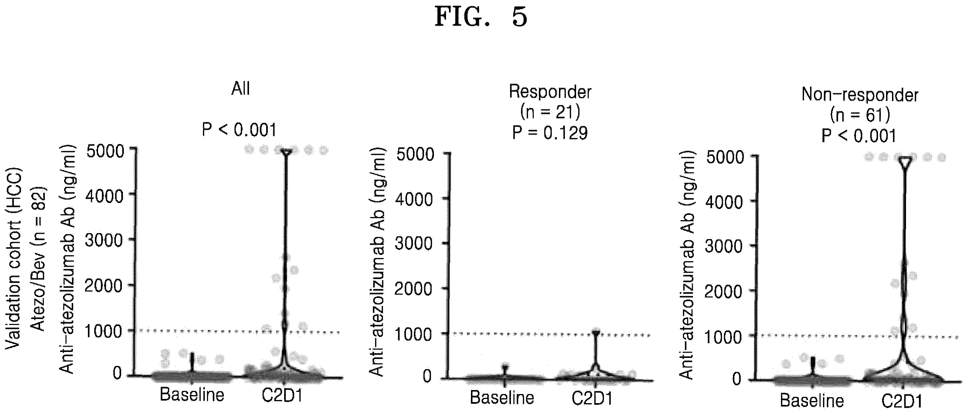

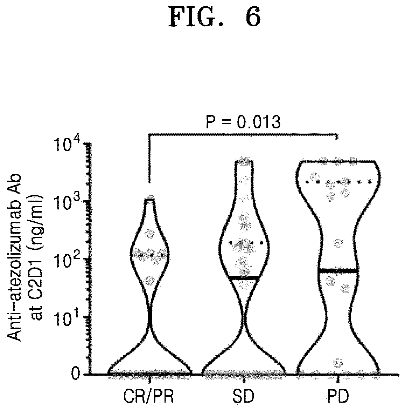

According to FIG. 3, in the advanced hepatocellular carcinoma discovery cohort, the atezolizumab ADA levels at C2D1 increased compared to baseline. The rise in ADA at C2D1 was prominent in the non-response group but not in the response group. Furthermore, as shown in FIG. 3, the ADA levels at C2D1 were significantly higher in patients with progressive disease (PD) compared to those with complete response (CR)/partial response (PR), or stable disease (SD). These results were consistently observed in the validation cohort. According to FIG. 5, ADA levels significantly increased at C2D1, and the non-response group showed a significant increase in ADA levels at C2D1. In addition, FIG. 6 indicates that ADA levels at C2D1 were higher in PD patients compared to those who experienced CR/PR in the validation cohort.

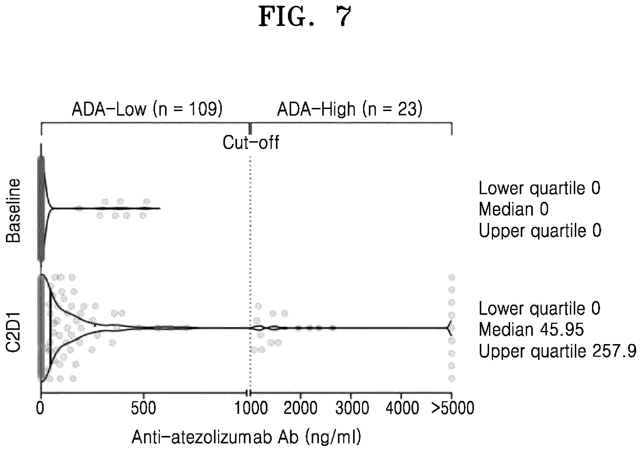

According to FIG. 7, Table 1, and Table 2, 17.4 % (n = 23) of advanced hepatocellular carcinoma patients treated with Atezo/Bev exhibited high levels of ADA at C2D1. Consistently, 13.3 % (n = 4) of advanced UC patients treated with atezolizumab also showed high ADA levels (≥1,000 ng/mL) at C2D1.

This confirms that ADAlevels were higher at C2D1 compared to baseline.

### Example 3. Establishment of Cut-off Value

Most patients did not develop atezolizumab ADA or had very low ADA levels at C2D1; however, some patients exhibited a very rapid and strong ADA response at C2D1. Baseline ADA values were <500 ng/mL in all patients, and to minimize false positives as much as possible, 1,000 ng/mL was set as the cut-off value to group patients into ADA-high (≥1,000 ng/mL) or ADA-low(<1,000 ng/mL).

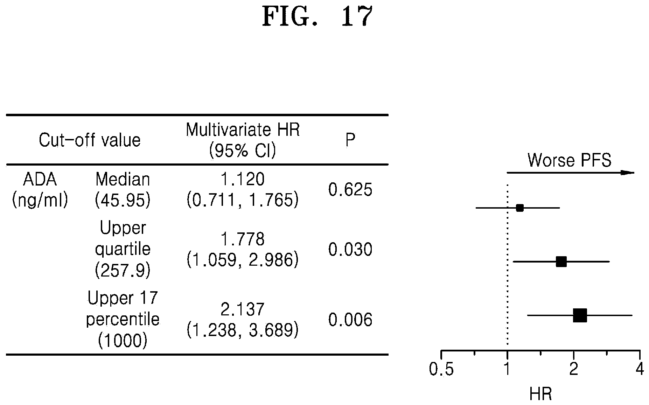

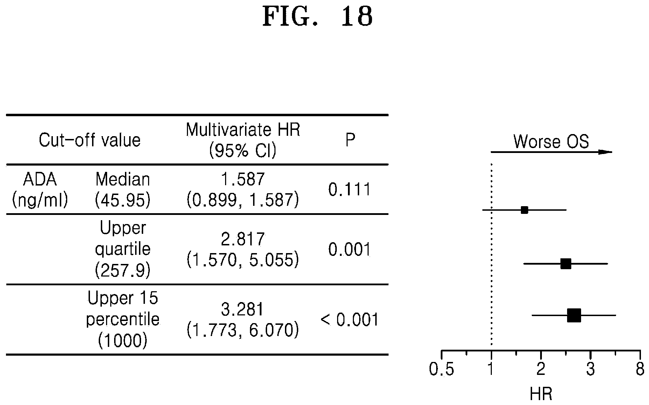

FIGS. 17 and 18 are meta-analysis forest plots showing PFS and OS according to various cut-offs for ADA positivity in the clinical outcome analysis of Example 4 below.

According to FIGS. 17 and 18, regarding PFS, the unfavorable clinical impact of ADA was significant when the cut-off value was sufficiently high

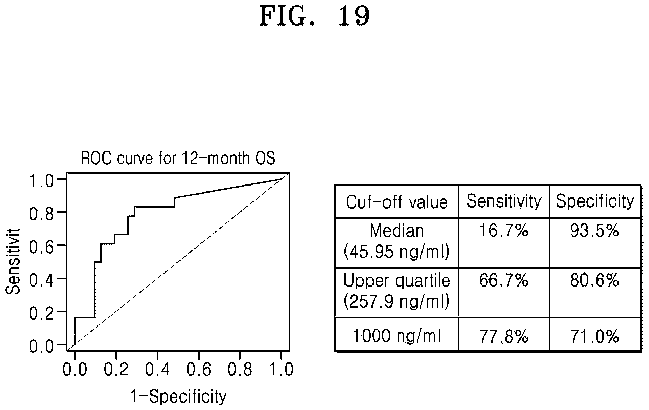

(≥1,000 ng/ml), and gradually decreased as the cut-off lowered to the upper quartile (257.9 ng/ml) or median value (45.95 ng/ml). Regarding OS, high ADA levels were associated with poor prognosis of atezolizumab-based therapy regardless of various cut-offs. ADA levels showed good predictive value for 12-month OS in the receiver operating curve analysis (AUC 0.78, 95 % CI, 0.63-0.82) of FIG. 19. The sensitivity and specificity at a cut-off ≥1,000 ng/ml were 77.8 % and 71 %, respectively.

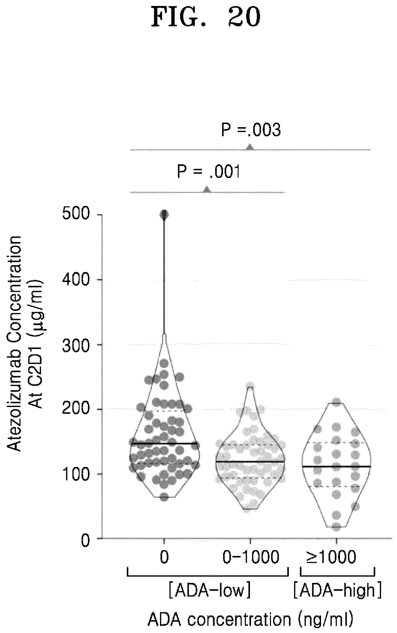

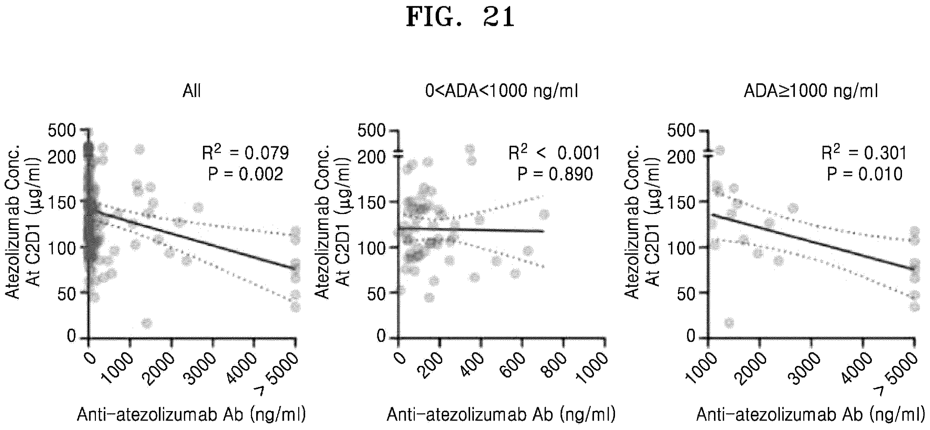

FIGS. 20 and 21 are graphs showing the correlation between serum atezolizumab and ADA concentrations in Example 5 below.

According to FIGS. 20 and 21, the inverse correlation between ADA concentration and serum atezolizumab concentration was pronounced in patients with ADA levels ≥1,000 ng/ml, but was not significant in those with ADA levels <1,000 ng/ml.

### Example 4. Clinical Outcome Confirmation for Atezolizumab-Based Therapy in Patients with High ADA Levels at C2D1

### Example 4-1. Analysis of Hepatocellular Carcinoma (HCC) Discovery Cohort, Independent Multicentric HCC Validation Cohort, and Urothelial Carcinoma (UC) Validation Cohort

To determine the clinical significance of high levels of atezolizumab ADA at C2D1 on the efficacy of Atezo/Bev therapy in patients with advanced hepatocellular carcinoma, the discovery cohort and independent multicentric validation cohort were analyzed using the methods of Reference Examples 1 to 4 and Reference Example 6. The clinical outcomes according to ADA status in patients with advanced UC treated with atezolizumab were also analyzed in the same manner.

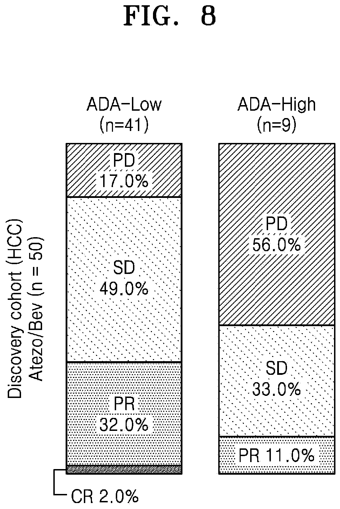

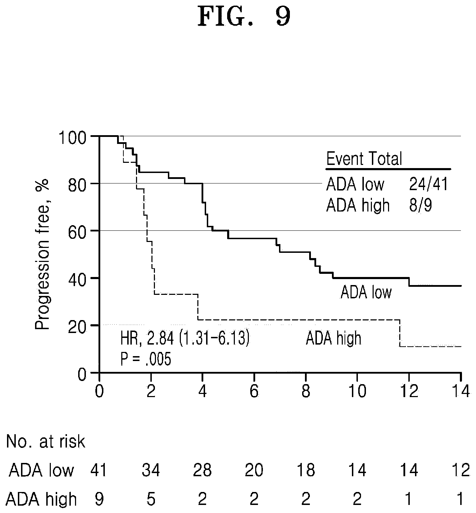

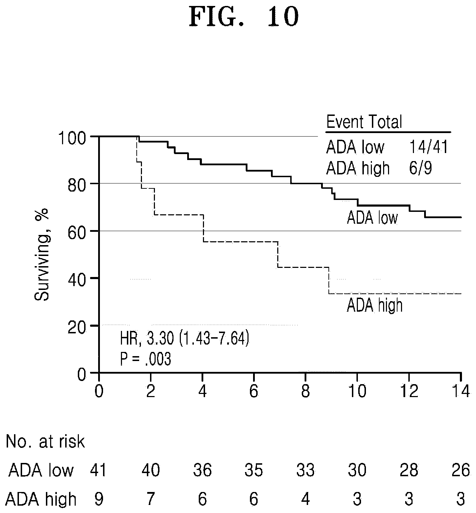

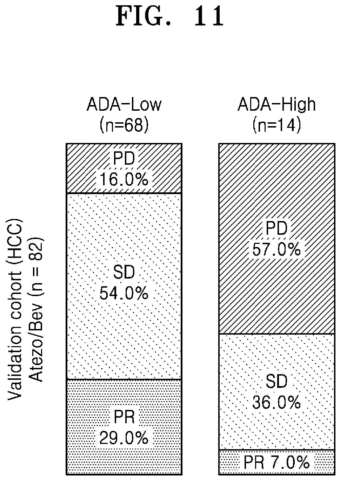

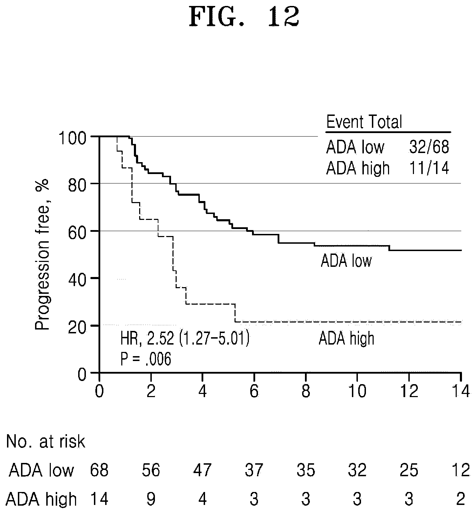

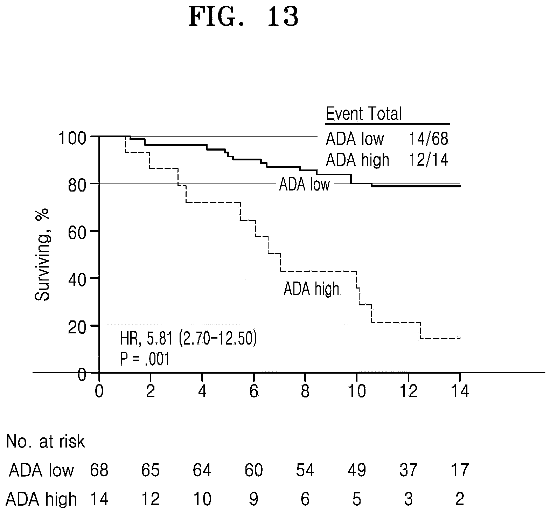

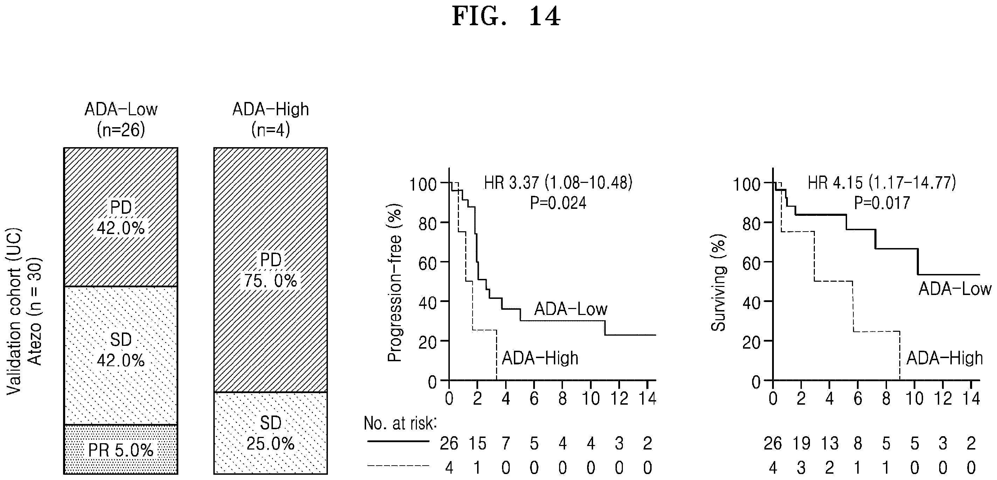

The analysis results of the hepatocellular carcinoma (HCC) discovery cohort are shown in FIGS. 8 to 10, the analysis results of the independent multicentric HCC validation cohort are shown in FIGS. 11 to 13, and the analysis of the urothelial carcinoma (UC) validation cohort is shown in FIG. 14.

According to FIGS. 8 to 10, in the discovery cohort, patients with high ADA levels at C2D1 had a lower ORR compared to patients with low ADA levels. Patients with high ADA levels also had significantly decreased PFS and OS compared to patients with low ADA levels.

According to FIGS. 11 to 13, the similar results were observed in the validation cohort. Patients with high ADA levels at C2D1 had a reduced ORR compared to patients with low ADA levels. PFS and OS were significantly poor in the ADA-high group compared to the ADA-low group.

According to FIG. 14, in the UC validation cohort, patients with high ADA levels at C2D1 also had lower ORR compared to patients with low ADA levels. Furthermore, UC patients with high ADA levels experienced poorer PFS and OS.

These results indicate that the occurrence of high levels of ADA at C2D1 is significantly associated with poor clinical outcomes of atezolizumab-based immunotherapy.

### Example 4-2. Multivariate Survival Analysis of ADA Status After Covariate Adjustment

Since various factors can confound the inferred clinical significance of ADA, the results were further validated through multivariate analysis. Analysis was performed using the method of Reference Example 6, using age, sex, ECOG performance status, Child-Pugh score, alpha-fetoprotein, macroscopic vascular invasion, extrahepatic spread, and neutrophil-to-lymphocyte ratio as covariates.

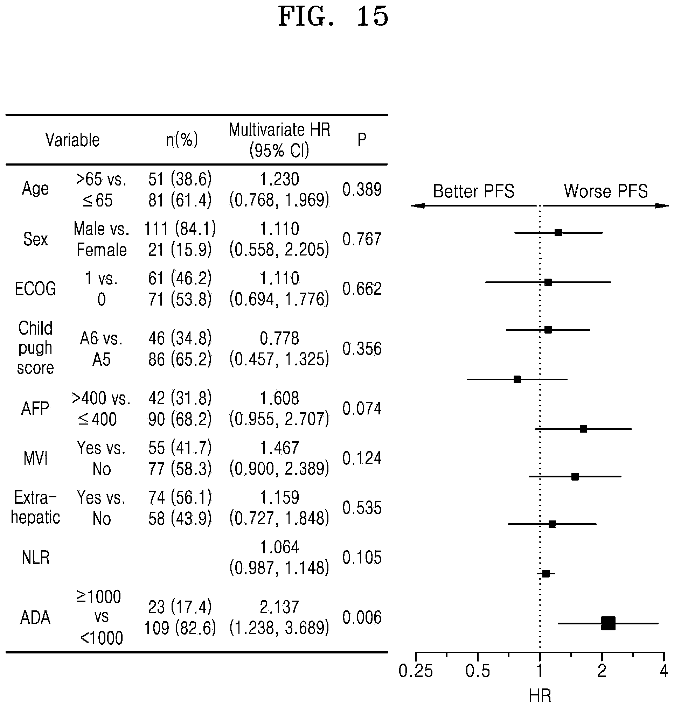

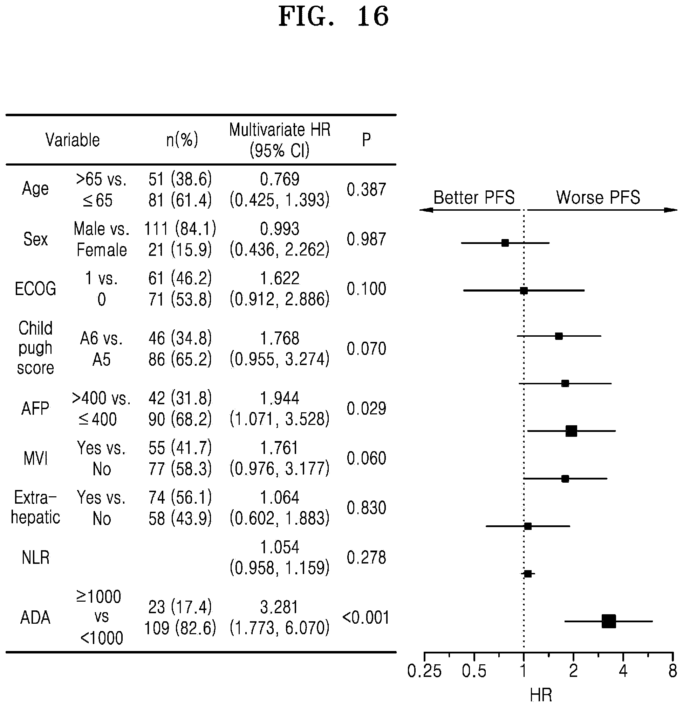

FIGS. 15 and 16 show the results of the multivariate analysis.

Even after adjustment for age, sex, ECOG performance status, Child-Pugh score, alpha-fetoprotein, macroscopic vascular invasion, extrahepatic spread, and neutrophil-to-lymphocyte ratio in the multivariate analysis, high ADA levels at C2D1 were independently associated with shorter PFS and OS.

Through these results, it was confirmed that patients with high ADA levels at C2D1 have poor clinical outcomes to atezolizumab-based therapy.

### Example 5. Confirmation of Correlation Between Reduced Serum Atezolizumab Concentrations, Impaired T Cell Responses, and ADA Levels

To further elucidate the impact of high ADA levels on the immunotherapeutic efficacy of atezolizumab, serum atezolizumab concentrations according to ADA status in patients with advanced hepatocellular carcinoma were evaluated. In addition, to investigate the effects of high ADA levels on T cell immunity, T cell proliferation responses and cytokine production were compared between the ADA-high group and the ADA-low group.

According to FIGS. 20 and 21, at C2D1, the atezolizumab concentration decreased by 23.5 % in patients with ADA values of 0 to 1,000 ng/mL, compared to patients who were ADA-negative (0 ng/mL). In addition, patients with ADA values of 1,000 ng/mL or higher showed a 29.8 % decrease in atezolizumab concentration at C2D1 compared to the ADA-negative group. At C2D1, the concentration of atezolizumab was inversely correlated with ADA levels in all patients.

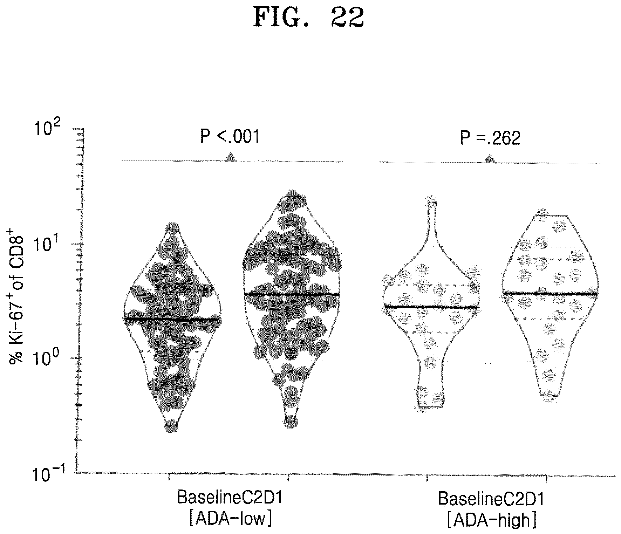

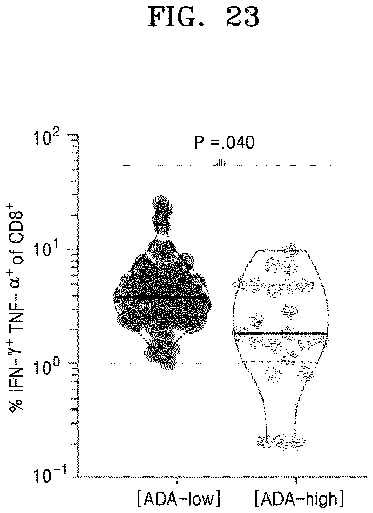

According to FIG. 22, the fraction of Ki-67+ CD8+ proliferating T cells significantly increased at C2D1 in the ADA-low group, but CD8+ T cell proliferation did not change significantly in the ADA-high group. Furthermore, in FIG. 23, CD8+ T cells from the ADA-high group showed decreased secretion of interferon-γ and tumor necrosis factor-α compared to the ADA-low group.

The inverse correlation between ADA concentration and serum atezolizumab concentration was pronounced in patients with ADA levels ≥1,000 ng/mL, but was not significant in those with ADA levels <1,000 ng/mL. Overall, high ADA levels were associated with decreased atezolizumab concentrations, reduced CD8+ T cell proliferation, and decreased secretion of effector cytokines from CD8+ T cells.

## Claims

1. A method of predicting therapeutic responsiveness to a biological drug, the method comprising measuring, from a biological sample isolated from a patient administered with the biological drug, a concentration of anti-drug antibodies specific to the biological drug.

2. The method of claim 1, wherein the biological sample is selected from the group consisting of blood, plasma, serum, bone marrow, tissue, cells, saliva, sputum, and urine.

3. The method of claim 1, wherein the biological sample is isolated 18 days to 24 days after initiation of treatment of the patient.

4. The method of claim 1, wherein the biological drug is a biological drug for treating a cancer.

5. The method of claim 4, wherein the cancer is urothelial carcinoma, non-small cell lung cancer, small cell lung cancer, triple-negative breast cancer (TNBC), hepatocellular carcinoma, colorectal cancer, lung cancer, glioblastoma multiforme, renal cell carcinoma, melanoma, bladder cancer, classical Hodgkin lymphoma (CHL), esophageal squamous cell carcinoma, esophageal cancer, gastric cancer, or head and neck squamous cell carcinoma.

6. The method of claim 1, wherein the biological drug is an antibody, a protein, a peptide, or a combination thereof.

7. The method of claim 6, wherein the antibody is an immune checkpoint inhibitor, a targeted anticancer agent, or a combination thereof.

8. The method of claim 7, wherein the immune checkpoint inhibitor is a CTLA4 inhibitor, a PD-L1 inhibitor, a PD-1 inhibitor, or a combination thereof.

9. The method of claim 7, wherein the immune checkpoint inhibitor is ipilimumab, tremelimumab, pembrolizumab, cemiplimab, nivolumab, durvalumab, atezolizumab, avelumab, or a combination thereof.

10. The method of claim 7, wherein the targeted anticancer agent is cetuximab, trastuzumab, ibritumomab, rituximab, brentuximab, alemtuzumab, bevacizumab, panitumumab, or a combination thereof.

11. The method of claim 1, wherein the concentration of anti-drug antibodies is measured by an enzyme-linked immunosorbent assay (ELISA).

12. The method of claim 1, further comprising determining the therapeutic responsiveness to the biological drug to be low when the measured concentration of anti-drug antibodies is greater than or equal to a cut-off value.

13. The method of claim 12, wherein the cut-off value is 500 ng/ml to 2,000 ng/ml.

14. A diagnostic kit for predicting therapeutic responsiveness to a biological drug, the diagnostic kit comprising a reagent capable of detecting anti-drug antibodies specific to the biological drug.

15. The diagnostic kit of claim 14, wherein a subject whose therapeutic responsiveness is to be predicted is a patient who has passed 18 days to 24 days after initiation of treatment with the biological drug.

16. The diagnostic kit of claim 14, wherein the reagent capable of detecting anti-drug antibodies is an antibody.

17. The diagnostic kit of claim 14, wherein the biological drug is a biological drug for treating a cancer.

18. The diagnostic kit of claim 17, wherein the cancer is urothelial carcinoma, non-small cell lung cancer, small cell lung cancer, triple-negative breast cancer (TNBC), hepatocellular carcinoma, colon cancer, lung cancer, glioblastoma multiforme, renal cell carcinoma, melanoma, bladder cancer, classical Hodgkin lymphoma (CHL), esophageal squamous cell carcinoma, esophageal cancer, gastric cancer, or head and neck squamous cell carcinoma.

19. The diagnostic kit of claim 14, wherein the biological drug is an antibody, a protein, a peptide, or a combination thereof.

20. The diagnostic kit of claim 19, wherein the antibody is an immune checkpoint inhibitor, a targeted anticancer agent, or a combination thereof.

21. The diagnostic kit of claim 20, wherein the immune checkpoint inhibitor is a CTLA4 inhibitor, a PD-L1 inhibitor, a PD-1 inhibitor, or a combination thereof.

22. The diagnostic kit of claim 20, wherein the immune checkpoint inhibitor is ipilimumab, tremelimumab, pembrolizumab, cemiplimab, nivolumab, durvalumab, atezolizumab, avelumab, or a combination thereof.

23. The diagnostic kit of claim 20, wherein the targeted anticancer agent is cetuximab, trastuzumab, ibritumomab, rituximab, brentuximab, alemtuzumab, bevacizumab, panitumumab, or a combination thereof.

24. The diagnostic kit of claim 14, wherein when a concentration of the anti-drug antibodies is equal to or greater than a cut-off value, the therapeutic responsiveness to the biological drug is determined to be low.

25. The diagnostic kit of claim 20, wherein the cut-off value is 500 ng/ml to 2,000 ng/ml.
